Europäisches Patentamt

(19) European Patent Office

Office européen des brevets

(11) Publication number: **0 118 634**
**A1**

## (12) EUROPEAN PATENT APPLICATION

(21) Application number: **83301437.6**

(22) Date of filing: **15.03.83**

(51) Int. Cl.³: **G 01 N 33/58**
**G 01 N 1/30**

(43) Date of publication of application:
**19.09.84 Bulletin 84/38**

(84) Designated Contracting States:
**AT BE CH DE FR GB IT LI LU NL SE**

(71) Applicant: **Davidson, Robert Stephen**
**496 Uppingham Road**
**Leicester LE5 6QB(GB)**

(71) Applicant: **Goodwin, Dean**
**Northampton Hall Bunhill Row**
**London EC1Y 8LJ(GB)**

(72) Inventor: **Davidson, Robert Stephen**
**496 Uppingham Road**
**Leicester LE5 6QB(GB)**

(72) Inventor: **Goodwin, Dean**
**Northampton Hall Bunhill Row**
**London EC1Y 8LJ(GB)**

(74) Representative: **Evans, David Charles et al,**
**F.J. CLEVELAND & COMPANY 40-43, Chancery Lane**
**London, WC2A 1JQ(GB)**

(54) **Mountant solution.**

(57) The invention relates to a mountant composition for use in retarding the fading of preparations stained for immunofluorescence with a fluorochrome which composition comprises one or more of sodium azide, sodium iodide and 1,4-diazobicyclo-[2,2,2]-octane in a suitable solvent carrier therefor.

EP 0 118 634 A1

- 1 -

MOUNTANT SOLUTION

DESCRIPTION

This invention relates to the retarding of immunoflurescence during microscopy. During the past two decades, the phenomenon of fading of fluorescein during excitation has been studied extensively in the course of the development of immunoflurescence methodology. The exposure of fluorescein stained samples to short wave (ultra violet) excitation, as provided, for example, by high pressure mercury vapour burners in common use, results in very rapid fading of fluorescein staining in tissue samples. The blue light emitted by quartz-halogen bulbs produces less rapid fading, but although these lower powered sources are entirely satisfactory with appropriate microscope accessories for viewing gross staining as seen for example with tissue sections in screening tests for auto-antibodies, more recent applications of immunofluorescence involving the staining of specific determinants on cell surfaces and the precise location of intracellular components and inclusions requires

- 2 -

maximum excitation by more powerful light sources. In consequence, the fading of fluorescein stained has become a major problem and efforts have been redoubled to find means of retarding or eliminating this fading.

It has recently been found that the addition of p-phenylenediamine (PPD) to the buffered glycerol used for mounting the stained preparation has a profound effect in retarding fading during subsequent microscopy. p-Phenylenediamine, however, is a material that is open to objection in routine use since it is a skin-sensitising agent, it is photo-sensitive and it undergoes oxidative degradation.

According to the present invention, there is provided a mountant composition for use in retarding the fading of preparations stained for immunofluorescence with a fluorochrome which composition comprises one or more of sodium azide, sodium iodide and 1, 4-diazobicylo-[2,2,2]-octane in a suitable solvent carrier therefor. The solvent carrier may be glycerol/phosphate buffered solution. The glycerol/ phosphate buffered solution may comprise 8 to 10 parts glycerol to one part of phosphate buffered solution and preferably has a pH within the range of 8 to 9. A further embodiment of the present invention provides a composition comprising a solvent of glycerol/phosphate buffered solution in the ratio of 9:1 volume:volume at a pH of 8.4 to 8.8 and a proportion of one or more of sodium azide, sodium iodide and 1,4-diazobicylo-[2,2,2]-octane in a concentration of at least $10^{-2}$M.

In a preferred embodiment of the present invention the standard solution for the mountant composition in accordance with the present invention may comprise 9 parts of glycerol, (typically an Analar grade, BDH) and one part phosphate buffered solution (PBS). The pH is preferably adjusted to match the fluorochrome. In the case of fluorescein isothiocyanate (FITC) the pH is adjusted to 8.6 by the addition of O.1m NAOH. When the retardant component is sodium azide or sodium iodide it may be dissolved in the PBS before addition to the glycerol. When the retardant component is 1,4-diazobicylo-[2,2,2]-octane (DABCO) it may be dissolved in the glycerol with gentle heat in a water bath before the addition of PBS.

- 4 -

Following is a description by way of example only and with reference to the accompanying drawings of methods of carrying the invention into effect.

In the drawings:-

Figure 1 is a graph showing the anode sensitivity against wavelengths for a photo multiplier designated R928.

Figure 2 is a graph showing the fading of ANA staining in glycerol; incident illumination with HPO 50 and HPO 100 high-pressure mercury burners and transmitted illumination with quartz-iodine lamp 100w; objective magnification x 16.

Figure 3 is a graph showing the effect on the fading rate of the addition of glycerol of $10^{-2}$M p-phenylenediamine (PPD) in which the illumination and magnification was as in Figure 2.

Figure 4 is a comparison of the effects of sodium azide, sodium iodide and DABCO each at a concentration of 2.5g per cent with HBO 50 incident illumination and objective magnification x16.

Figure 5 is a graph showing the effect of PVA and PVP, each at 2.5g per cent illumination and magnification as in Figure 4.

Figure 6 is a graph showing the effect on the fading rate of adding PPD ($10^{-2}$M) and DABCO (2.5g per cent) at magnification x40 (n.a. 0.95) with the HBO 50 lamp and comparison of bleaching rates obtained with the slides mounted in the plain glycerol exposed to HBO 50 and HBO 100 lamps at x40 magnification incident illumination.

Figure 7a is a graph showing the rates of photo-fading of fluorescence intensity of a solution of FITC conjugate in PBS under oxygen and argon.

Figure 7b is a graph showing the fluorescence fading rates of FITC conjugate in $H_2O$ and $D_2O$ under oxygen.

Figure 8a is a graph showing the fluorecence fading rates of FITC conjugate in PBS in the presence of NaI, DABCO or $NaN_3$ under argon.

Figure 8b is a graph corresponding to Figure 8a showing the same fading rates, but under oxygen.

Figure 9a is a graph showing the flurescence fading rates of FITC conjugate in buffered glycerol containing NaI, DABCO or $NaN_3$ under argon.

Figure 9b is the same as Figure 9a showing the same fading rates under oxygen.

MATERIALS AND METHODS

1.   Anti-nuclear Staining

The IF technique employed was based on cyostat sections (6 um) of rat liver which were treated with a pool of ANA positive sera, diluted 1:10, known to give a mixed diffuse and speckled nuclear staining pattern; after washing in PBS (0.15M NaCl in 0.01M phosphate pH 7.4) the sections were stained for 30 minutes with anti-human Ig-FITC conjugate (Wellcome Reagents) diluted 1:160 and after further washing mounted in glycerol mixtures prepared as described below. Use of multispot slides enabled replicate series of measurements to be made on previously un-exposed test areas.

2.  Preparation of Mounting Fluids

The standard solution contained 9 parts of glycerol (Analar grade, BDH) and 1 part PBS. The pH was adjusted to 8.6 (optimum for FITC fluorescence emission - Nairn 1976) by the addition of 0.1M NaOH. Test substances were usually dissolved in the PBS before it was added to the glycerol with the exception of DABCO (v.infra) which was solubilised directly in the glycerol with gentle heat in a water-bath before addition of PBS; the addition of aminopiperidine (which is basic) necessitated adjustment of pH with 0.1M HCl. The following compounds were tested: sodium azide (Aldrich), soldium iodide (Hopkin and Williams) polyvinyl alcohol (Cytochemical Co.) polyvinyl pyrrolidone (Sigma), 4-amino-2,2',6,6'-tetramethylpiperidine (Ciba-Geigy), indole (Koch-Light), gramine (Aldrich) NN dimethylaniline (Aldrich), diphenylamine (Aldrich), and 1,4-diazobicyclo-[2,2,2]-octane (DABCO) (Ralph N Emmanuel).

3. Microscopy

A Zeiss Photomicroscope II equipped for incident illumination was employed with high-pressure mercury burners HBO 100 (stablised) and HBO 50 (unstablised); the excitation wavelength transmittted by the primary filter was 450-490 nm and the barrier filter was a long pass LP 520. Measurements were also made using transmitted darkfield illumination with a 100W quartz-iodine lamp run at 12 volts, Balzer FITC-3 primary filter and Kodak Wratten 12 secondary filter. The FITC-3 filter was supplemented with Pilkington LP1 (red absorbing). All light sources were previously un-used.

4. Fluorometry

a) Equipment

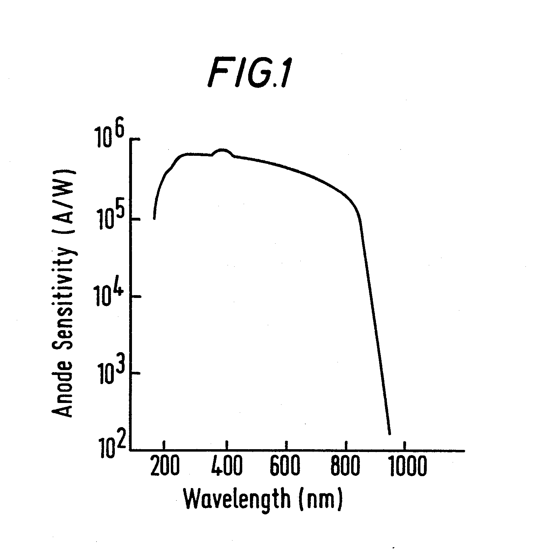

This was based on the Zeiss SF microscope photometer and comprised a photomultiplier showing high response at the wavelength of FITC emission (R928) (Fig 1) attached to the microscope tube head. The latter incorporated a measuring diaphragm. The photomultiplier was connected to a power supply incorporating a 4 digit voltmeter. The opening of the measuring diaphragm corresponded to an area of $10^{-2}$ $mm^2$, determined with a haemocytometer and a x16 Planachromat objective, which included approximately 20 hepatocytes. Results obtained were therefore not subject to possible variation due to differences in staining intensity of individual nuclei and represent the total light emitted by representative areas of the sections. An apochromatic x40 objective having a high numerical aperture (0.95) was employed in later experiments for comparative purposes as described below.

b)    Procedure

The gain control of the voltmeter was adjusted to give a reading of about 80% of maximum with the dark current at zero and the stained section in focus. These settings were not altered during a session of comparative measurements. A previously un-exposed section was then placed in position with the microscope lamp shutter closed, focussing was checked on opening the shutter, the stage moved to an adjacent field and the voltmeter reading taken; simultaneously a timer was started. Readings were taken every 15 seconds for the first minute and at one minute

- 10 -

intervals for a total of five minutes. Blank readings were taken in the same way of an unstained section mounted in the same medium as the stained slide. These blank readings were found to be crucial to the study, accounting for as much as 25% of the values obtained on stained sections. The sections employed for blank measurements were washed in parallel with the stained sections as it was found that cryostat sections mounted directly showed approximately twice the luminosity of washed sections. A further source of irrelevant "blank" values was eliminated by removing the sub-stage condenser assembly from the incident microscope as this component was found to enhance readings by back reflection.

5. <u>Calculation of Results</u>

Blank readings showed a very slight decay amounting to a few (less than 5) units on the voltmeter scale, mainly in the first 15 seconds' exposure; these were substracted from the corresponding readings obtained on stained sections which were then expressed as a percentage of the initial reading and plotted against time on linear ordinates. At least two sets of readings were taken using virgin fields for each and means values calculated. Each set of comparisons shown was obtained in a single run.

## 6. Solution Phase Studies

Fluorescence spectra were recorded using a Perkin-Elmer MPF-4 fluorescence spectrophotometer, at an excitation wavelength of 490 nm.

Irradiation of the solution was performed in a stoppered fluorimeter cell (10 mm x 45 mm) containing a small, glass covered, metal bar. The cell was mounted on a magnetic stirrer and light from a 75W high-pressure Xenon lamp, at a distance of 25 cm, focussed on to the cell. A semi-circular metallic reflector was positioned behind the cell, and the solution was continually stirred throughout the irradiation.

A stock solution of the anti-human Ig-FITC conjugate used in the ANA system was prepared in phosphate buffered saline (0.15M NaCl in 0.01M phosphate pH 7.4) at a concentration of 3mg in 10 ml. 0.5 ml aliquots of this solution were diluted to 5 ml using the appropriate solvent distilled water, deuterium oxide (Goss), phosphate-buffered saline (as above), or glycerol (BDH, AR Grade). After thorough mixing, 3 ml of the solution were transferred to a fluorimeter cell and the mixture flushed with either oxygen or argon for 5 minutes and then stoppered.

The fluorescence spectrum of the solution was recorded prior to, and on cessation of the illumination period (usually 2 1/2, 5, 10, 20 and 30 minutes). The fluorescence intensity (at 520 nm) after each irradiation was calculated as a percentage of the initial fluorescence intensity prior to irradiation. By this method the photo-fading of the fluorescence of the FITC conjugate in solution was conveniently monitored. Similar experiments were performed in the presence of DABCO, $NaN_3$ and NaI (0.1M respectively). Readings were adjusted for thermal degradation by subtracting the measurements obtained on control solutions kept in the dark during the irradiation period.

The efficiency with which the additives (DABCO, $NaN_3$ and NaI) quenched the fluorescence of the FITC conjugate in phosphate-buffered saline solution was assessed by determining the Stern-Volmer quenching

- 13 -

constants (Cox and Kemp 1971). This was achieved by diluting 2.5 ml of the stock solution of the FITC conjugate to 25 ml with phosphate buffered saline, then taking 5 ml aliquots of this solution and adding different amounts of the quencer (between O and 0.25 M). 3 ml of the prepared solution was transferred to a fluorimeter cell and the fluorescence spectrum recorded, measuring the intensity of fluorescence at 520 nm. In this way, plots of Io/I versus concentration of quencher gave the Stern-Volmer quenching constant according to the equation:

$$\frac{I_o}{I} = 1 + K_{sv}(Q)$$

where: $I_o$ = Intensity of fluorescence in the absence of quencher.

$I$ = Intensity of fluorescence in the presence of quencher at a concentration $(Q)$.

Each Stern-Volmer plot was subject to least squares analysis by computer.

A microsecond flash photolysis apparatus (Applied Photophysics Ltd) was used to detect the triplet state of fluorescein (Lindqvist 1960). The effect of additives such as DABCO, $NaN_3$ and NaI upon the triplet states was monitored at 550nm.

RESULTS

1. Fading in Standard Buffered Glycerol

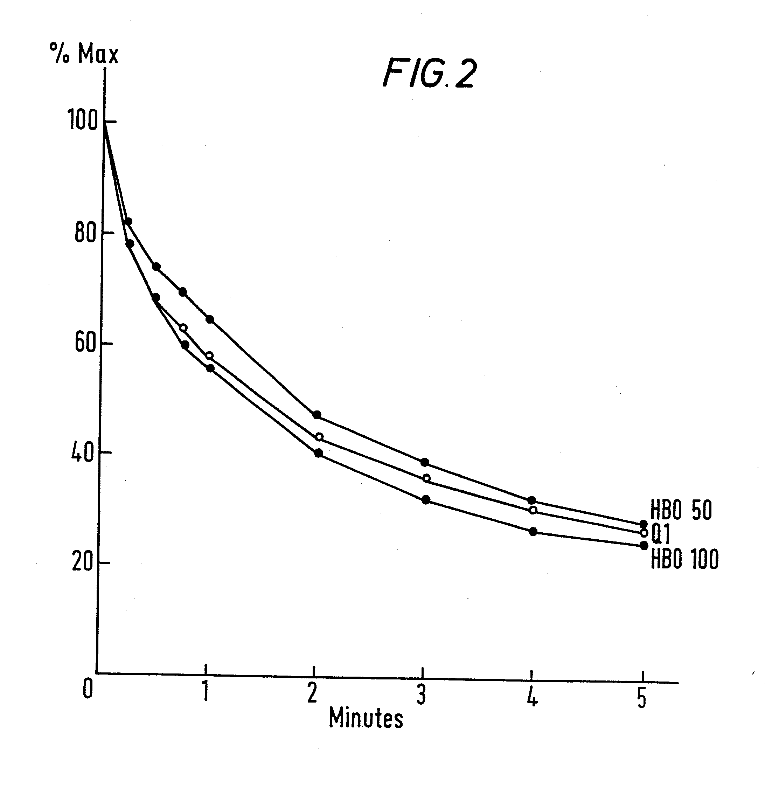

A comparison of the rates of fading obtained with the three light sources at low magnification (x16) is shown in Figure 2. The more potent HBO 100 burner induced a slightly more rapid fading by incident illumination than the HBO 50 and the Q1 lamp employed in the transmitted light system was intermediate.

2. Retardation by PPD

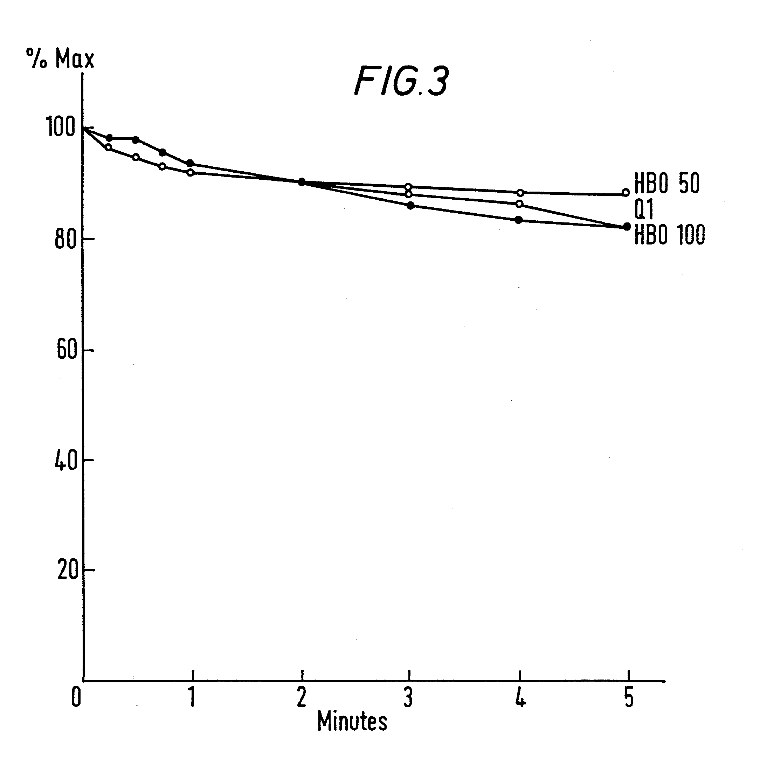

Addition of the amine at a concentration of $10^{-2}$M resulted in a much slower decay with overlapping of the curves during the first two minutes (Figure 3).

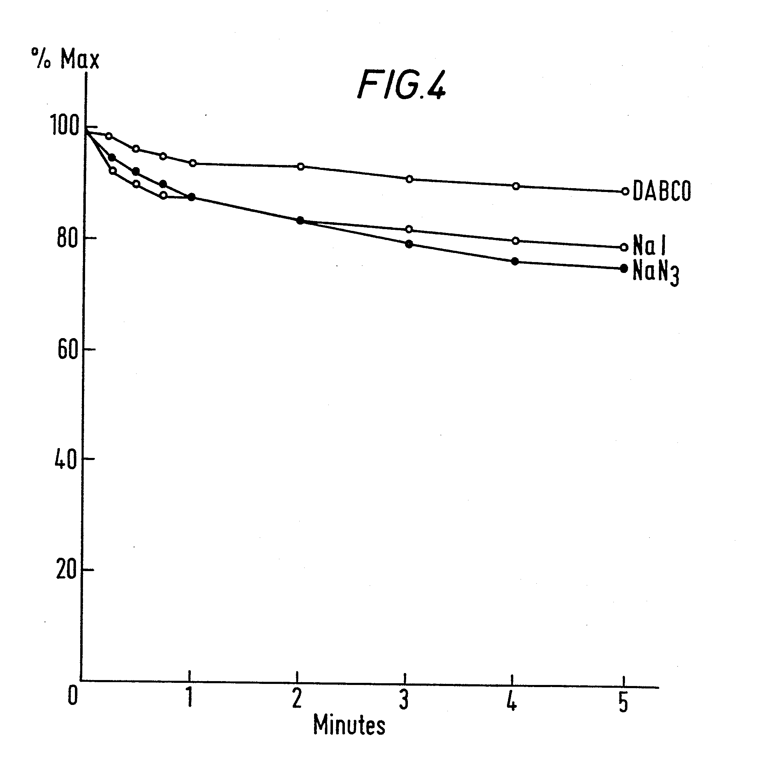

3. Effects of Sodium Azide, Sodium Iodide and DABCO Preliminary tests indicated that these additives did produce retardation, but that a higher concentration than $10^{-2}$M was required for this to be at a useful level. The results obtained with a final

- 15 -

concentration of 2.5g per cent are shown in Figure 4 for the widely-employed HBO 50 incident system. It is evident that at this concentration all the materials have a significant effect.

3. Other Additives

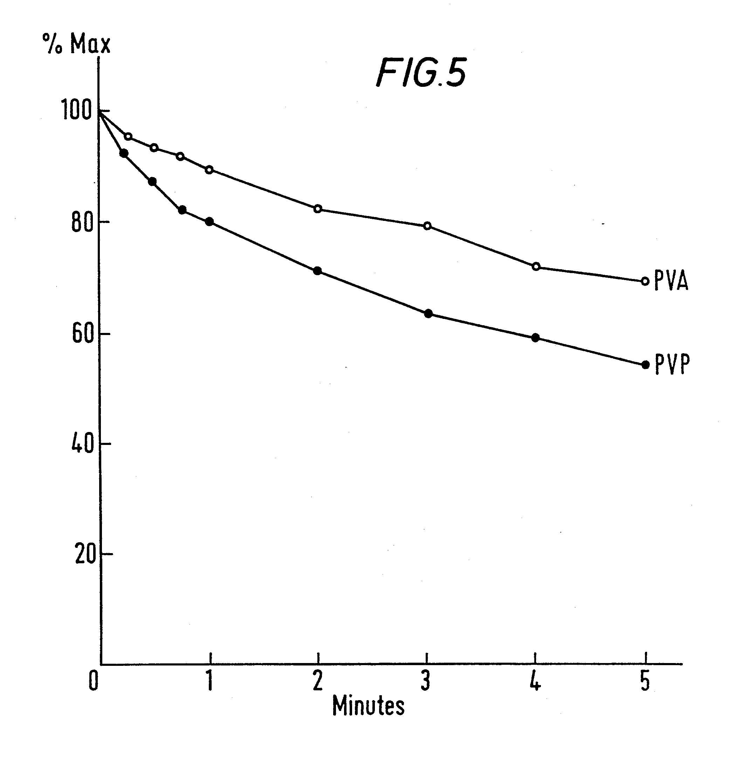

Initially, promising results were obtained with aminopiperidine. However, this substance appeared to be unstable since subsequent findings were inconsistent and further investigation of it was therefore discontinued. Other additives which retarded fading at high concentration were polyvinyl alcohol and (less effectively) polyvinylpyrrolidone. Their performance is shown in Figure 5. Indole, gramine, NN-dimethylalanine and diphenylamine all showed no retardation when screened at a concentration of $10^{-2}$M.

5. Effect of Increased Magnification

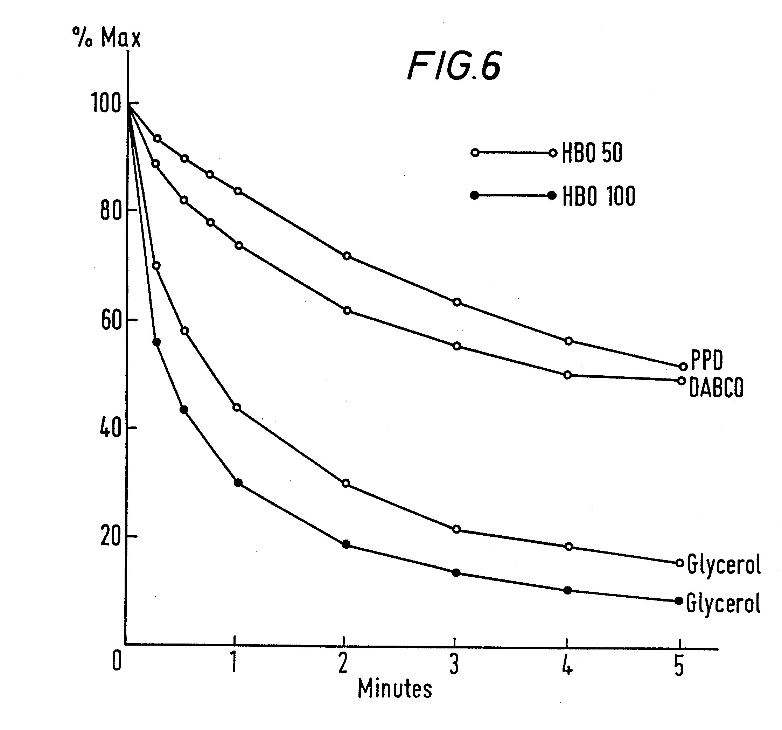

Since in practice higher magnification than x16 is usually employed measurements were repeated using the dry x40 apochromat n:a 0.95 in the incident light system. When used with the HBO 100 lamp this high performance objective significantly accelerated fading of a section mounted in standard buffered glycerol (Figure 6). When PPD ($10^{-2}$M) and DABCO 2.5g per cent were re-tested at the higher magnification with the more conventional HBO 50 lamp they both showed a reduced but still marked anti-fading effect (Figure 6). In further experiments using a x63 planachromat oil immersion objective n.a. 1.4 with the HBO 100 lamp we recorded a fluorescence half-life of 6 seconds in preparations mounted in plain buffered glycerol.

6.   Solution Phase Studies

Solutions of the FITC conjugate were found to be unstable at room temperature and it was therefore necessary to correct all the measurements made on irradiated solutions to allow for thermal degradation.

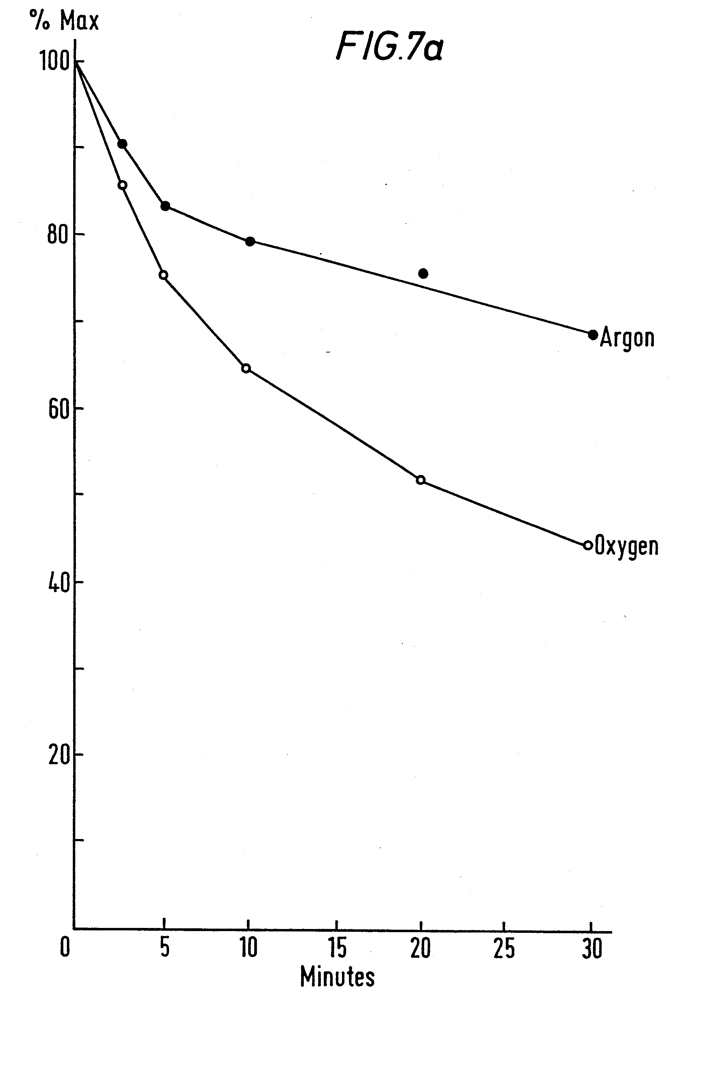

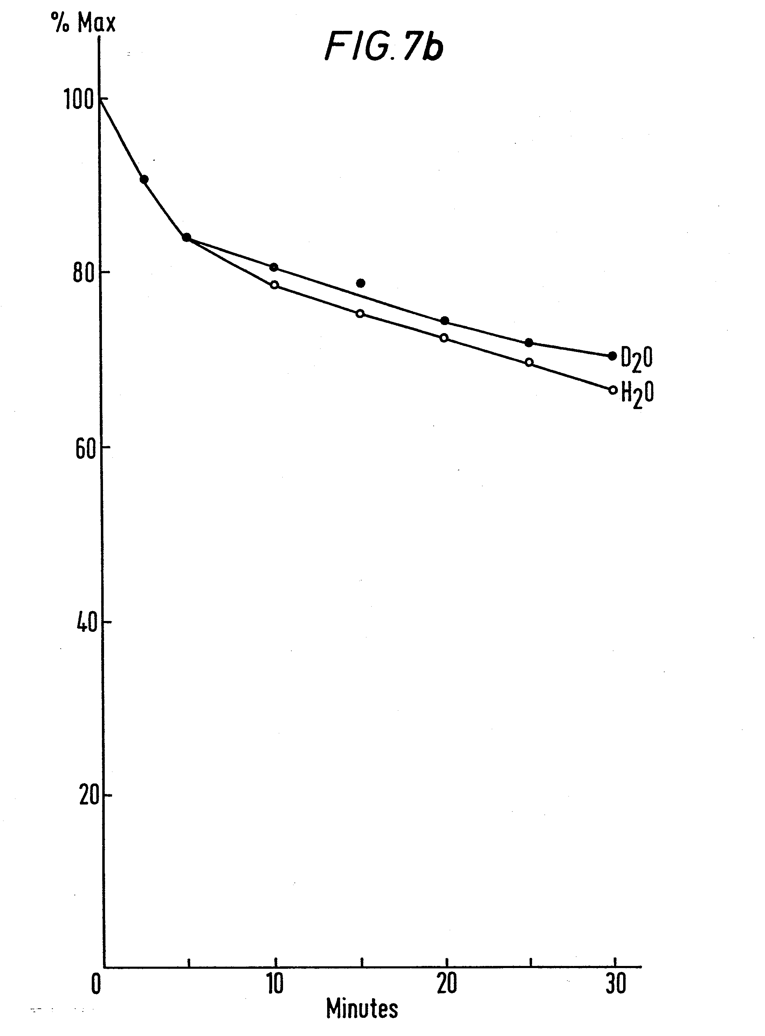

Irradiation of the FITC conjugate in phosphate buffered saline solution in the presence or absence of oxygen led to bleaching of the fluorescence. Bleaching was slightly faster when oxygen was present (Figure 7a). The rate of bleaching was measured for solutions of FITC conjugate in oxygenated distilled water and oxygenated deuterium oxide, and little difference found between the two systems, i.e. there was no solvent isotope effect upon the bleaching process (Figure 7b).

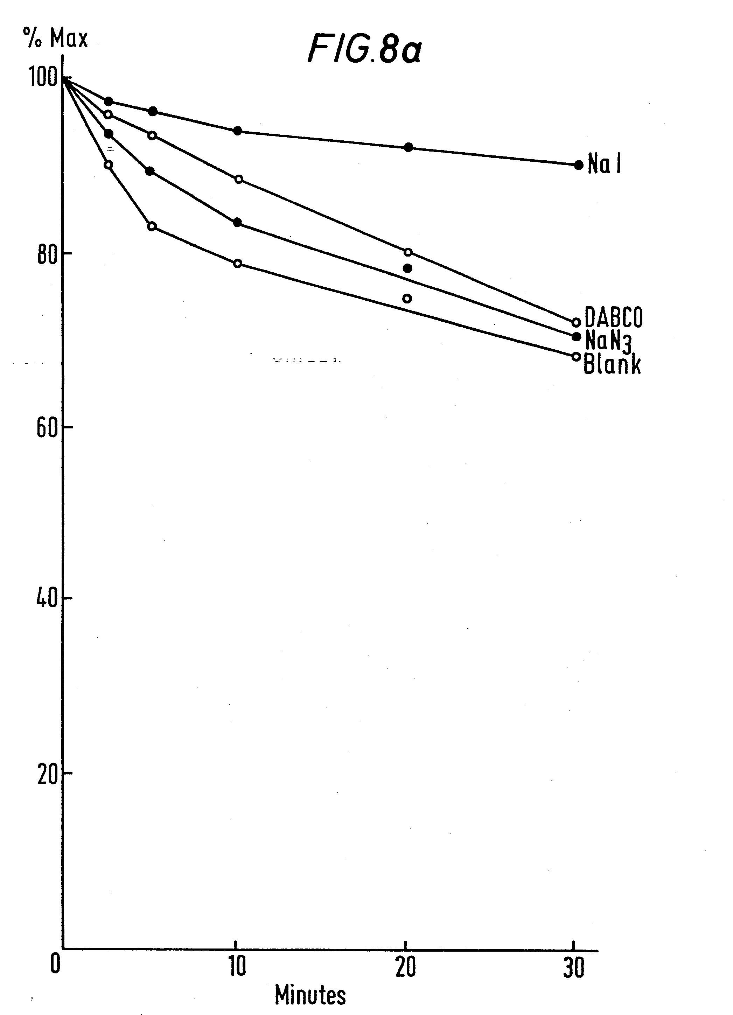

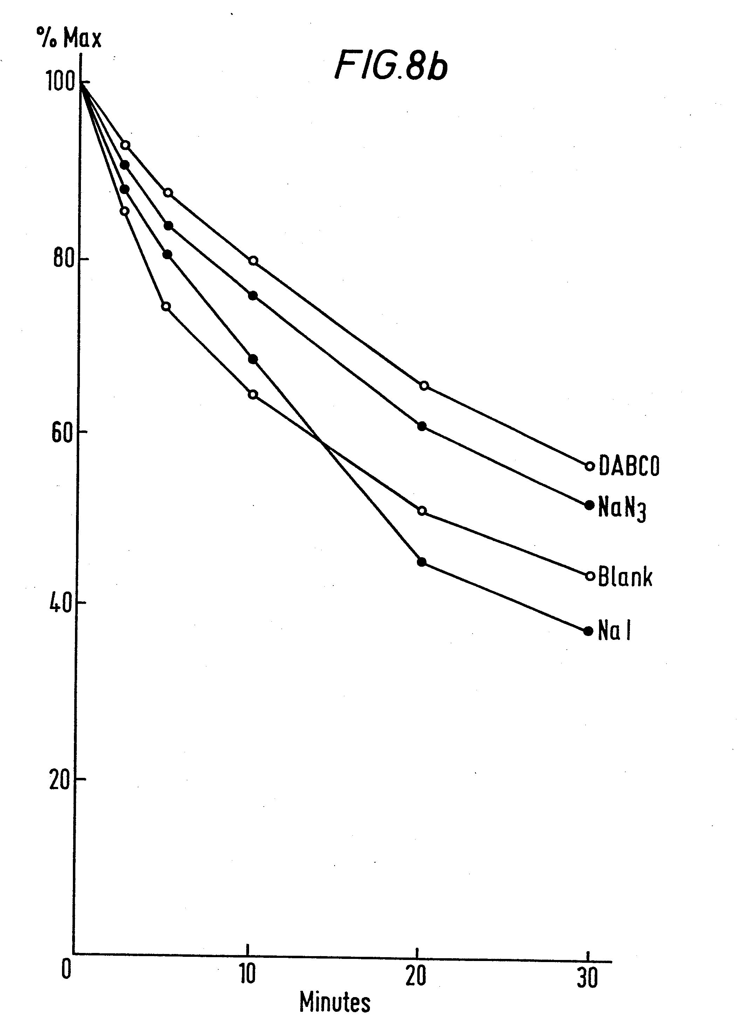

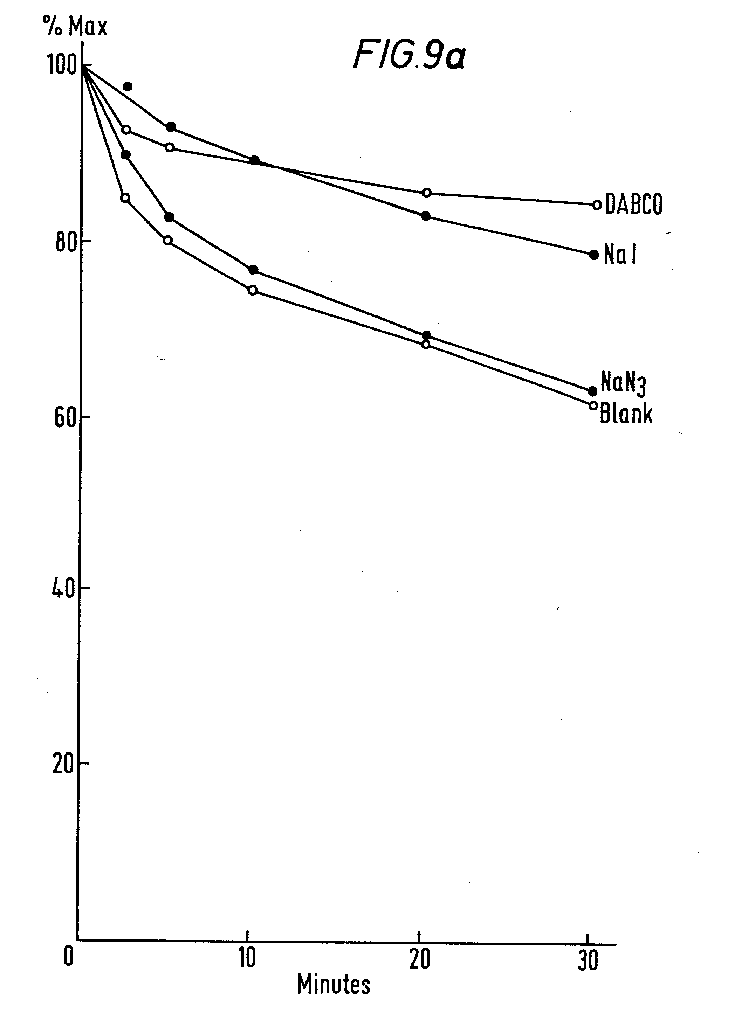

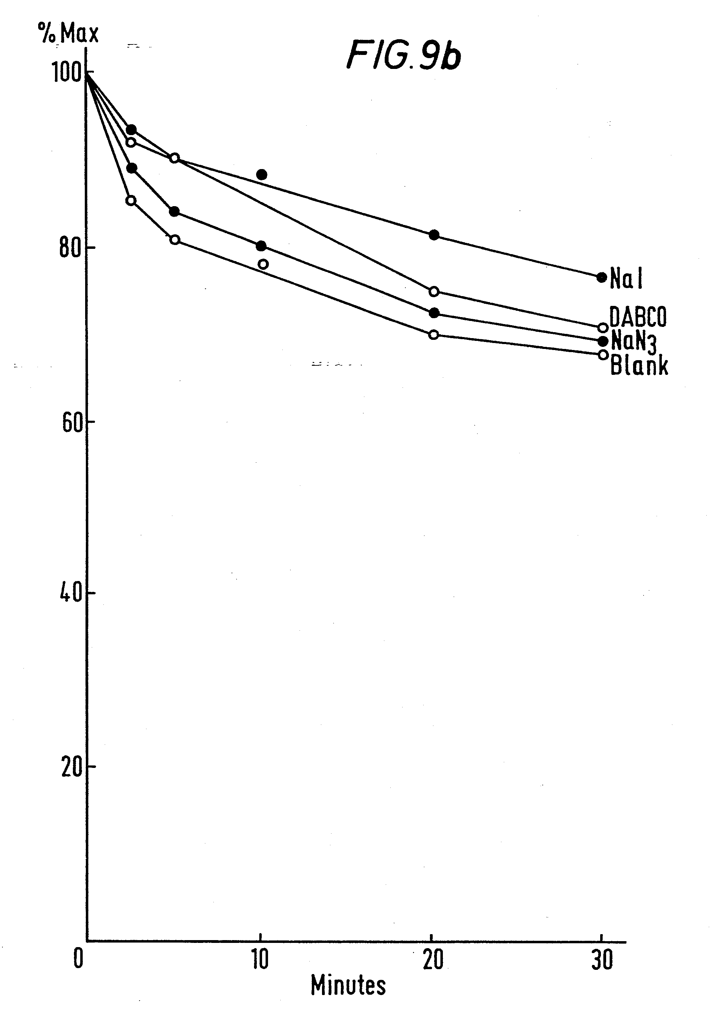

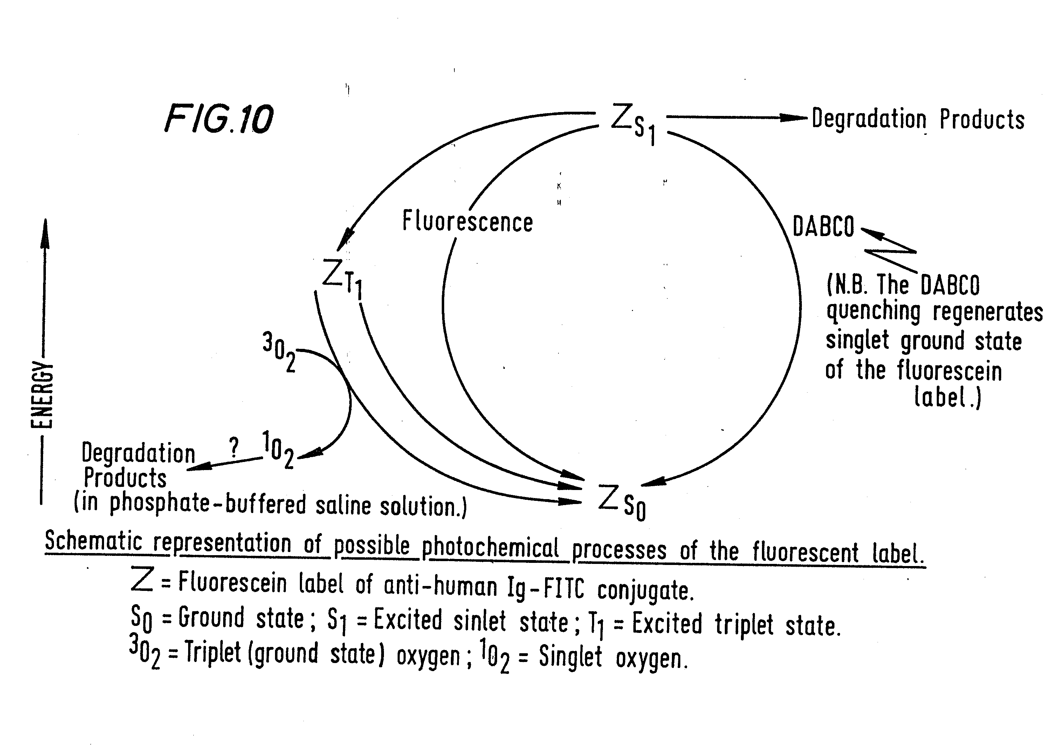

When the bleaching was studied using glycerol solutions containing buffered FITC conjugate, the bleaching was less rapid and the presence of oxygen had no noticeable effect. DABCO, $NaN_3$ and NaI all retarded the bleaching process in both the aqueous buffered solution (Figures 8a, 8b) and in the buffered glycerol solution (Figures 9a, 9b).

Stern-Volmer quenching constants for the quenching of the FITC conjugate in phosphate buffered solution by DABCO, $NaN_3$ and NaI were $3.95 \pm 0.24$, $2.54 \pm 0.34$ and $3.77 \pm 0.22$ respectively.

Flash photolysis studies showed that the triplet state of fluorescein is not quenched by DABCO, $NaN_3$ or NaI.

Following are examples of mountant compositions in accordance with the present invention.

- 18 -

Example 1

Glycerol/phosphate buffered saline for applications using labelled tissue slices on microscope slides.

2.5gm of 1,4-diazabicyclo-[2,2,2]-octane (DABCO) in 100ml of a glycerol/phosphate buffer solution (9:1v/v) was prepared in the manner described above. The phosphate buffered solution comprises 0.87g sodium chloride, 0.024g of calcium dihydrogen phosphate, 0.189g of disodium hydrogen phosphate dissolved in 100ml of distilled water. The mountants are used in the manner described above.

Example 2

Provides for a phosphate buffered saline mountant for applications using suspensions of labelled cells, for example in the technique of fluorescein antibody cell sorting. 2.5g of DABCO is dissolved in 100ml of phosphate buffered saline of example 1. The mountant solution is used in the manner described above.

- 1 -

CLAIMS

1.    A mountant composition for use in retarding the fading of preparations stained for immunofluorescence with a fluorochrome which composition comprises one or more of sodium azide, sodium iodide and 1,4-diazobicyclo-[2,2,2]-octane in a suitable solvent carrier therefor.

2.    A composition as claimed in claim 1 characterised in that the solvent is glycerol/ phosphate buffered solution.

3.    A composition as claimed in claim 1 characterised in that the solvent is a phosphate buffered solution.

4.    A composition as claimed in claim 1 characterised in that the solvent is a glycerol/ phosphate buffered solution comprising 8 to 10 parts glycerol to 1 part of phosphate buffered solution having a pH of 8 to 9.

5. A composition as claimed in claim 1 characterised by comprising a solvent of glycerol/phosphate buffered solution in the ratio of 9:1 v/v with a pH of 8.4 to 8.8 and a proportion of one or more of sodium azide, sodium iodide and 1,4-diazobicyclo-[2,2,2]-octane in a concentration of at least $10^{-2}$M.

6. A composition as claimed in any one of the preceeding claims wherein the fluorochrome is fluorescein isothiocyanate.

7. A composition as claimed in claim 6 wherein the pH is 8.6.

# FIG.1

FIG.2

FIG.3

0118634

3/13

4/13

0118634

FIG.4

FIG.5

FIG.7a

0118634

FIG.7b

0118634

FIG.8a

0118634

FIG.8b

FIG.9a

FIG.9b

*FIG.10*

Schematic representation of possible photochemical processes of the fluorescent label.

$Z$ = Fluorescein label of anti-human Ig-FITC conjugate.

$S_0$ = Ground state; $S_1$ = Excited sinlet state; $T_1$ = Excited triplet state.

$^3O_2$ = Triplet (ground state) oxygen; $^1O_2$ = Singlet oxygen.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 98, no. 11, 14th March 1983, page 245, no. 85548f, Columbus, Ohio, USA G.D. JOHNSON et al.: "Fading of immunofluorescence during microscopy: a study of the phenomenon and its remedy" & J. IMMUNOL. METHODS 1982, 55(2), 231-242 * Whole abstract *  | 1,2,6 | G 01 N 33/58 G 01 N 1/30 |
| A | GB-A-1 538 018 (D.L. BLAKESLEE) * Pages 1,2; examples 5-11; claims 1,5 * | 1,3,4 | |

TECHNICAL FIELDS SEARCHED (Int. Cl. 3)

G 01 N

The present search report has been drawn up for all claims

| Place of search | Date of completion of the search | Examiner |
|---|---|---|
| THE HAGUE | 17-11-1983 | GRIFFITH G. |